# NEUE EUROPÄISCHE PATENTSCHRIFT

(11) **EP 1 164 999 B2**
(45) Veröffentlichungstag und Bekanntmachung des Hinweises auf die Entscheidung über den Einspruch: **29.09.2010**
(45) Hinweis auf die Patenterteilung: 13.10.2004
(21) Anmeldenummer: 00916894.9
(22) Anmeldetag: 03.03.2000
(51) Int. Cl.: A61F 13/15

(54) **HYGIENEARTIKEL ZUM EINMALIGEN GEBRAUCH**
SINGLE-USE HYGIENE ITEMS
ARTICLE HYGIENIQUE A USAGE UNIQUE

(30) Priorität: 27.03.1999 DE 19914037
(43) Veröffentlichungstag der Anmeldung: 02.01.2002
(73) Patentinhaber: Paul Hartmann Aktiengesellschaft, 89522 Heidenheim (DE)
(72) Erfinder: BITTERHOF, Andreas, D-89564 Nattheim-Auernheim (DE)
(74) Vertreter: Friz, Oliver
(86) Internationale Anmeldenummer: PCT/EP2000/001824
(87) Internationale Veröffentlichungsnummer: WO 2000/057827

(56) Entgegenhaltungen:
- DE-A- 3 035 902
- GB-A- 2 003 925
- US-A- 3 918 433
- US-A- 5 823 953
- US-A- 5 876 389

## Beschreibung

Die Erfindung betrifft einen Hygieneartikel zum einmaligen Gebrauch, wie eine Windel, eine Inkontinenzvorlage oder eine Damenbinde, mit einem Körperflüssigkeiten aufnehmenden und haltenden Saugkörper und mit einer Analysevorrichtung für die Körperflüssigkeit (siehe GB-A-2 003 925).

Es sind Hygieneartikel bekannt, welche Indikatorstreifen aufweisen, die bei Benässung mit Urin einen Farbwechsel ausführen und so den Zustand einer Durchnässung visuell wahrnehmbar anzeigen. Da es sich bei diesem Indikatorstreifen im weitesten Sinne um eine Analysevorrichtung für die Körperflüssigkeit handelt, wurde im Oberbegriff von einem Hygieneartikel mit einer Analysevorrichtung ausgegangen.

Es besteht jedoch bei Hygieneartikeln nicht nur die Notwendigkeit, deren Einnässungszustand zu überwachen und im erschöpften Zustand des Hygieneartikels anzuzeigen, sondern es besteht grundsätzlich das Bedürfnis, beispielsweise Urin im Hinblick auf seine Beschaffenheit zu untersuchen. Daher wird mit der EP 0 438 482 B1 auch vorgeschlagen, in einen Urinauffangbeutel eine Analysevorrichtung mit urinexponierten Indikatoren vorzusehen. Insbesondere handelt es sich dabei um Indikatoren, welche in den Urinauffangbeutel gelangenden Urin auf dessen pH-, Nitrit-, Leukozyt-, Glucose- und Elektrolytwerte untersuchen. Die Indikatoren sind zunächst weiss und nehmen nach Benetzung mit dem zu untersuchenden Urin eine Farbe ein. In saurem Medium wird sich beispielsweise ein Abschnitt eines pH-Wert-Indikators gelb färben und im alkalischen Medium wird er sich blau färben. Es sind als Kontrollkarten bezeichnete Analysevorrichtungen beschrieben, die unter einem jeweiligen Indikatorabschnitt ein Farbenfeld mit mehreren Farben aufweisen, anhand derer ein Benutzer die Farbreaktion des betrachteten Indikators zu einem Wert zuordnen kann. Dieser Wert ist in einer weiteren Zeile unterhalb des Farbenfelds vorgesehen.

Nach der Lehre dieser Druckschrift ist ein jeweiliges Indikatorfeld auf der urinexponierten Seite mit einer Membran abgedeckt, die eine aus sehr kleinen Löchern gebildete Perforation aufweist. Diese Membran bildet eine Art Dosiervorrichtung und lässt den Urin nur langsam zum Indikatorfeld gelangen. Auf diese Weise wird verhindert, dass in den noch leeren Auffangbeutel eintretender Vorlaufurin mit hoher Keimkonzentration eine überkritische Anzeige bewirkt, die vom Mittelstrahlurin normalerweise nicht ausgelöst werden kann. Ebenso kann auch der vom Mittelstrahl abweichende Resturin nicht allein anzeigebestimmend wirken. Infolge des verhältnismässig verlangsamten Durchtritts durch die Membran wird der zu untersuchende Urin "gut gemischt" auf das Indikatorfeld geleitet. In vorteilhafter Weiterbildung der Analysevorrichtung beschreibt diese Druckschrift, dass die Indikatorsubstanzen des Indikatorfeldes in einem Material eingebettet sind, das bei Feuchtigkeit quillt. Auf diese Weise kann beim Aufquellen ein Druck des Materials gegen die Membran aufgebaut und dadurch der Urindurchgang weiter erschwert werden. Auf diese weise wird einem Ausschwemmen der farbbildenden Indikatorsubstanzen entgegengewirkt, und die quasi eingefrorenen Anzeigewerte können auch nach vielen Stunden unverfälscht abgelesen werden.

Wann immer Analysevorrichtungen zum Einsatz kommen, besteht das Bestreben oder sogar die Notwendigkeit, sicherzustellen, dass die zu untersuchenden Substanzen möglichst unverfälscht mit den meßsensitiven Mitteln, also mit den Indikatoren der Analysevorrichtung, in Kontakt gelangen, um ein wechselwirkungsfreies, möglichst unverfälschtes Messergebnis zu gewährleisten.

Daher wurde die Analysevorrichtung gemäss der genannten EP 0438482 B1 auch in einem an einen Blasenkatheder anschliessenden Urinableitsystem in Form eines Urinauffangbeutels angeordnet.

Dies wird von Patienten, deren Körperflüssigkeit einer Untersuchung unterzogen werden soll, verständlicherweise als unangenehm empfunden. Im übrigen ist es medizinisch aufwendig. Bei inkontinenten Personen besteht auch nicht die Möglichkeit, die zu untersuchende Körperflüssigkeit zu Untersuchungszwecken in ein Gefäss abzugeben.

Aus GB-A-2 003 925 ist ein Hygieneartikel bekannt, bei dem im Saugkörpermaterial eine Kapsel mit Öffnungen vorgesehen ist, um Flüssigkeit aus dem Saugkörpermaterial eintreten zu lassen. Im Inneren der Kapsel kann dann eine Signalreaktion ablaufen.

Hiervon ausgehend liegt der vorliegenden Erfindung die Aufgabe zugrunde, die Anwendung einer an sich bekannten und in der EP 0 438 482 B1 beschriebenen Analysevorrichtung zu erweitern, im besonderen die Verwendung eines an einen Blasenkatheder anschliessenden Urinableitsystems nicht erforderlich zu machen.

Zur Lösung dieser Aufgabe wird ein Hygieneartikel mit den Merkmalen des Anspruchs 1 vorgeschlagen.

Die erfindungsgemässe Ausbildung des Hygieneartikels hat den Vorteil, dass zu untersuchende Körperflüssigkeit nicht durch Wechselwirkung mit eventuell vorhandenen Saugkörpermaterialien des Hygieneartikels bzw. durch Herauslösen von die Analysereaktion beeinflussenden Substanzen aus dem Saugkörpermaterial beeinflusst und damit das Messergebnis verfälscht wird. So umfasst der Saugkörper moderner Hygieneartikel üblicherweise sogenannte superabsorbierende Polymermaterialien, die ein Vielfaches ihres Gewichts an Flüssigkeit binden. Diese Substanzen wirken sich jedoch auf die Messung einzelner Bestandteile verfälschend aus. Dadurch, dass die Analysevorrichtung durch das erwähnte Trennmittel im wesentlichen flüssigkeitsdicht von dem Saugkörper des Hygieneartikels separiert ist, wird sichergestellt, dass Urin, welcher zuvor infolge der Wechselwirkung mit den Saugkörpermaterialien im Hinblick auf die Analysereaktion verfälscht wurde, nicht zu der Analysevorrichtung gelangt und dort analysiert wird.

Nach einer weiteren Ausführungsform des erfindungsgemässen Hygieneartikels sind Randbereiche dieses Einsatzes auf dessen Oberseite eingeschlagen.

Der Einsatz ist vorteilhafterweise von einer flüssigkeitsundurchlässigen Folie begrenzt, die zugleich das Trennmittel bildet.

Nach der Erfindung ist der Einsatz im wesentlichen flächenbündig mit der körperzugewandten Oberseite des Saugkörpers angeordnet.

Grundsätzlich könnte die Analysevorrichtung in an sich beliebiger Weise durch das Trennmittel gegen den Saugkörper separiert vorgesehen werden. In Weiterbildung der Erfindung wird jedoch vorgeschlagen, dass die Analysevorrichtung in Anlage an eine Seite des Trennmittels angeordnet ist. Bei Beaufschlagung des Hygieneartikels mit Körperflüssigkeit wird diese üblicherweise der Schwerkraft folgend bis zu dem Trennmittel gelangen und dort in Kontakt mit der Analysevorrichtung treten.

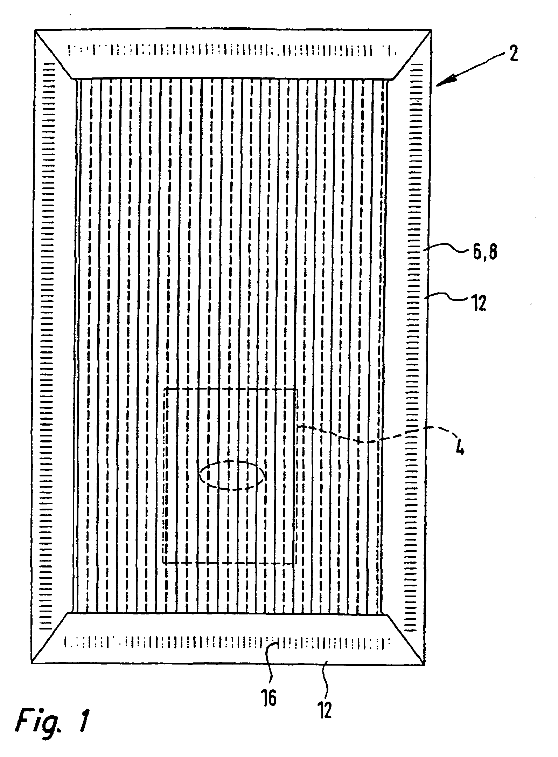

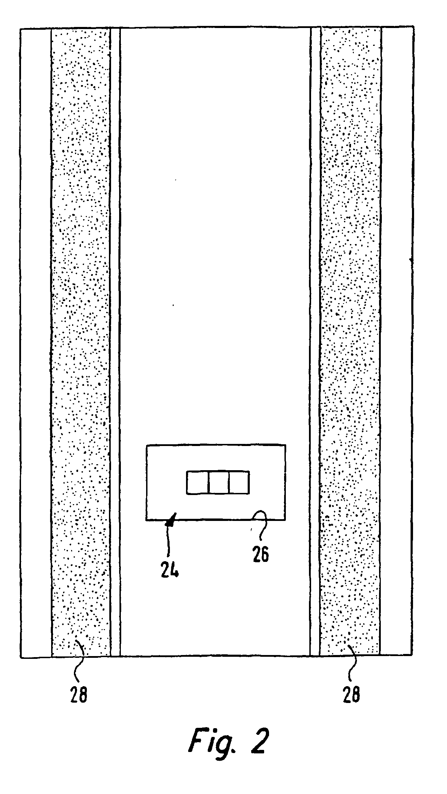

Ein an sich üblicher bekannter Hygieneartikel, beispielsweise eine Windel, eine Inkontinenzvorlage oder eine Damenbinde, kann nach der vorliegenden Erfindung mit einer Analysevorrichtung zur Messung und Kontrolle der Zusammensetzung von Körperflüssigkeiten ausgebildet werden. In den Fig. 1 bis 3 ist eine erste Ausführungsform eines in einen üblichen Hygieneartikel integrierbaren Einsatzes 2 mit einer darin aufgenommenen Analysevorrichtung 4 vorgesehen. Der Einsatz 2 ist von einer flüssigkeitsundurchlässigen Folie 6 gebildet, welche beim Einsetzen des Einsatzes 2 in einen Hygieneartikel oder in die Oberseite eines Saugkörpers eines Hygieneartikels ein Trennmittel 8 bildet, welches die Analysevorrichtung 4 gegen die Saugkörpermaterialien des Hygieneartikels separiert und verhindert, dass Körperflüssigkeiten, insbesondere Urin, die zunächst von einem Benutzer des Hygieneartikels in den Saugkörper des Hygieneartikels gelangen, von dort die Analysevorrichtung 4 erreichen können. saugkörpermaterialien moderner Hygieneartikel enthalten nämlich Stoffe, beispielsweise superabsorbierende Polymermaterialien, welche Substanzen in die Körperflüssigkeit abgeben und dadurch deren Beschaffenheit, insbesondere bei der Messung der pH-, Nitrit-, Leukozyt-, Glucose- oder Elektrolytwerte, verändern. Das in der Analysevorrichtung ermittelte Messergebnis wäre daher fehlerhaft.

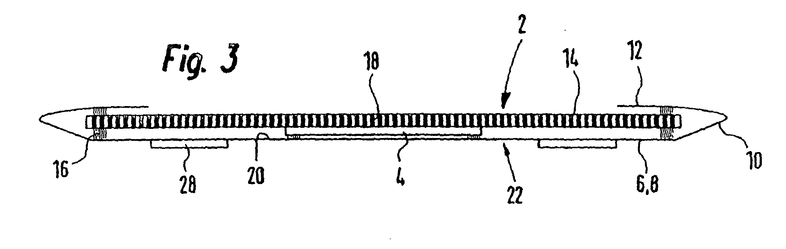

Mit der Erfindung wird daher vorgeschlagen, dass die Analysevorrichtung durch das Trennmittel 8 von den Saugkörpermaterialien des Hygieneartikels getrennt oder separiert wird. Hierfür wird, wie bereits erwähnt, der Einsatz 2 verwendet. Der Einsatz 2 weist zur Körperseite eines Benutzers hin nach oben kragende Seitenwände 10 auf. Randbereiche 12 des Einsatzes, die sich an die Seitenwände 10 anschliessen, sind auf eine Oberseite 14 nach innen umgeschlagen. Die Randbereiche 12 sind in dieser Position fixiert, und zwar über eine in den Fig. 1 und 3 angedeutete Verklebung oder Verschweissung 16.

Der Einsatz 2 umfasst neben der Analysevorrichtung 4 eine Flüssigkeitsaufnahme- und -transportschicht 18 zur Aufnahme und zum Transport von von einem Benutzer des Hygieneartikels unmittelbar abgegebener Körperflüssigkeit. Diese Schicht 18 ist vorteilhafterweise von einem Material gebildet, das eine zur Aufnahme der Körperflüssigkeit ausreichende Aufnahmekapazität aufweist und dabei gleichzeitig hinreichende Kapillarität besitzt, um einen Transport der Körperflüssigkeit innerhalb der Schicht 18 zu gewährleisten, und zwar ohne dabei Substanzen freizusetzen, die einen Einfluss auf das in der Analysevorrichtung 4 zu ermittelnde Messergebnis haben können. Es werden vorteilhafterweise zellulosische Werkstoffe, also vorzugsweise luftgelegter natürlicher Zellstoff, angewandt, aber auch ein durch Bikomponentenfasern etwa aus Polyäthylen, Polypropylen verfestigtes Viskosevlies wäre denkbar und vorteilhaft.

Die Speicherschicht 18 dient der Aufnahme und dem Transport der Körperflüssigkeit zur Analysevorrichtung 4, sie gewährleistet aber auch, dass eine hinreichende Menge der zu analysierenden Körperflüssigkeit erfasst und zwischengespeichert wird.

Wie aus Fig. 3 ersichtlich ist, ist die Analysevorrichtung 4 in Anlage an eine Innenseite 20 der das Trennmittel 8 bildenden flüssigkeitsundurchlässigen Folie 6 angeordnet. Die Folie 6 ist in diesem Bereich durchsichtig ausgebildet, so dass eine Einsichtnahme auf die Analysevorrichtung 4 von der Unterseite 22 des Einsatzes 2 möglich ist. Zum Ablesen der Analysevorrichtung 4 weist diese eine optische Anzeigeeinheit 24 auf, die durch den durchsichtigen Bereich 26, der eine Art Sichtfenster bildet, einsehbar ist. Die Anzeigeeinheit 24 umfasst vorteilhafterweise Farbindikatoren, die entsprechend der Konzentration einer zu bestimmenden Substanz eine Farbreaktion ausführen und zur Anzeige bringen, die dann durch Zuordnung zu vorgegebenen Farbfeldern eine Auswertung des Messergebnisses durch den Benutzer der Analysevorrichtung 4 möglich macht.

Der Einsatz 2 ist in die Oberseite eines Saugkörpers eines Hygieneartikels eingelassen. Nach Gebrauch des Hygieneartikels wird der gesamte Einsatz 2 von dem Hygieneartikel gelöst, der dann weggeworfen werden kann. Der Einsatz 2 kann dann auf bequeme Weise zusammengefaltet werden, wobei die das Trennmittel 8 bildende Folie 6 die Aussenseite bildet. Über den erwähnten durchsichtigen Bereich 26 ist eine Einsichtnahme bzw. Ablesung der Anzeigeeinheit 24 der Analysevorrichtung 4 möglich.

Zum lösbaren Befestigen des Einsatzes 2 an dem Hygieneartikel weist dieser auf seiner im Gebrauch körperabgewandten Seite haftende oder klebende Befestigungsmittel 28 auf, die im dargestellten Fall beispielhaft als in Längsrichtung des Einsatzes 2 durchgehende Streifen vorgesehen sind.

## Patentansprüche

1. Hygieneartikel zum einmaligen Gebrauch, wie Windel, Inkontinenzvorlage, Damenbinde, mit einem superabsorbierende Polymermaterialien enthaltenden Körperflüssigkeiten aufnehmenden und haltenden Saugkörper und mit einer Analysevorrichtung für die Körperflüssigkeit, **dadurch gekennzeichnet,**
**dass** die Analysevorrichtung (4) an mit der zu analysierenden Körperflüssigkeit beaufschlagbaren Stelle des Hygieneartikels vorgesehen ist und über ein Trennmittel (8) im wesentlichen flüssigkeitsdicht von dem Saugkörper separiert ist, so dass von einem Benutzer abgegebene und zu analysierende Körperflüssigkeit direkt zu der Analysevorrichtung (4) gelangen kann, in den Saugkörper gelangte Körperflüssigkeit jedoch von dem Trennmittel (8) zurückgehalten und von der Analysevorrichtung (4) ferngehalten wird, und dass das Trennmittel (8) einen die Analysevorrichtung (4) beinhaltenden Einsatz (2) in dem Saugkörper bildet, der wannenförmig ausgebildet ist und dass der Einsatz (2) im wesentlichen flächenbündig mit der körperzugewandten Oberseite (14) des Saugkörpers angeordnet ist.

2. Hygieneartikel nach Anspruch 1, **dadurch gekennzeichnet, dass** der Einsatz (2) zur Körperseite hin kragende Seitenwände (10) aufweist.

3. Hygieneartikel nach einem der vorstehenden Ansprüche, **dadurch gekennzeichnet, dass** Randbereiche (12) des Einsatzes (2) auf dessen Oberseite (14) eingeschlagen sind.

4. Hygieneartikel nach einem der vorstehenden Ansprüche, **dadurch gekennzeichnet, dass** der Einsatz (2) von einer flüssigkeitsundurchlässigen Folie (6) begrenzt ist.

5. Hygieneartikel nach einem der vorstehenden Ansprüche, **dadurch gekennzeichnet, dass** die Analysevorrichtung (4) in Anlage an eine Seite (20) des Trennmittels (8) angeordnet ist.

6. Hygieneartikel nach einem der vorstehenden Ansprüche, **dadurch gekennzeichnet, dass** die Analysevorrichtung (4) eine optische Anzeigeeinheit aufweist.

7. Hygieneartikel nach Anspruch 5 oder 6, **dadurch gekennzeichnet, dass** die Seite (20) des Trennmittels (8), gegen die die Analysevorrichtung (4) anliegt, zumindest im Bereich der Analysevorrichtung (4) durchsichtig ausgebildet ist, so dass eine visuelle Ablesung der Anzeigeeinheit möglich ist.

8. Hygieneartikel nach einem der vorstehenden Ansprüche, **dadurch gekennzeichnet, dass** das die Analysevorrichtung beinhaltende Trennmittel (8) von dem Hygieneartikel lösbar ist.

9. Hygieneartikel nach einem der vorstehenden Ansprüche, **dadurch gekennzeichnet, dass** das die Analysevorrichtung (4) beinhaltende Trennmittel (8) über klebende oder haftende Befestigungsmittel (28) an dem Hygieneartikel lösbar befestigt ist.

10. Hygieneartikel nach einem der vorstehenden Ansprüche, **dadurch gekennzeichnet, dass** der die Analysevorrichtung beinhaltende Einsatz (2) eine Flüssigkeitsaufnahme- und - transportschicht (18) aufweist, welche die Körperflüssigkeit zu der Analysenvorrichtung leitet.

11. Hygieneartikel nach Anspruch 10, **dadurch gekennzeichnet, dass** die Flüssigkeitsaufnahme- und - transportschicht (18) Zellstofffasern ohne Zusatz superabsorbierender Polymermaterialien umfasst.

## Claims

1. Single-use hygiene article, such as a diaper, incontinence article, sanitary napkin, having an absorbent element for absorbing and retaining bodily fluid containing superabsorbent polymer materials and having an analysis device for the bodily fluid, **characterized in that** the analysis device (4) is disposed at an area of the hygiene article on which the bodily fluid to be analyzed can impinge and is separated in an essentially fluid-tight manner from the absorbent element by a separating means (8), so that bodily fluid passed by a user to be analyzed can reach the analysis device (4) directly, but bodily fluid that has penetrated to the absorbent element is retained by the separating means (8) and kept away from the analysis device (4), and that the separating means (8) forms an insert (2) in the absorbent element containing the analysis device (4), which insert (2) is dish-shaped and that the insert (2) is located essentially flush-mounted with the upper side (14) of the absorbent element facing the body.

2. Hygiene article in accordance with claim 1, wherein the insert (2) has sidewalls (10) extending up on the side facing the body.

3. Hygiene article in accordance with one of the preceding claims, wherein edge areas (12) of the insert (2) are folded in on the upper side (14) of the insert (2).

4. Hygiene article in accordance with one of the preceding claims, wherein the insert (2) delineated by a fluid-impermeable film (6).

5. Hygiene article in accordance with one of the preceding claims, wherein the analysis device (4) is positioned adjacent to one side (20) of the separating means (8).

6. Hygiene article in accordance with one of the preceding claims, wherein the analysis device (4) has a visual display unit.

7. Hygiene article in accordance with claim 5 or 6, wherein the side (20) of the separating means (8) which is adjacent to the analysis device, is made transparent, at least in the area of the analysis device (4), so that a visual reading of the display unit is possible.

8. Hygiene article in accordance with one of the preceding claims, wherein the separating means (8) containing the analysis device is separable from the hygiene article.

9. Hygiene article in accordance with one of the preceding claims, wherein the separating means (8) containing the analysis device (4) is fastened detachably to the hygiene article by means of adhesive or touch-sensitive means of attachment (28).

10. Hygiene article in accordance with one of the preceding claims, wherein the insert (2) containing the analysis device has a fluid absorbing and transport layer (18) which transfers the bodily fluid to the analysis device.

11. Hygiene article in accordance with claim 10, wherein the fluid absorbing and transport layer (18) comprises cellulose fibers without the addition of superabsorbent polymer materials.

## Revendications

1. Article d'hygiène à usage unique, tel que couche pour bébé, couche pour incontinent, serviette hygiénique, comportant un corps absorbant contenant de matériaux polymères superabsorbants qui reçoit et conserve les liquides corporels et un dispositif d'analyse pour le liquide corporel, **caractérisé en ce que** le dispositif d'analyse (4) est prévu à un endroit de l'article d'hygiène qui peut recevoir le liquide corporel à analyser et est séparé de manière substantiellement étanche aux liquides du corps absorbant au moyen d'un élément de séparation (8) de telle manière qu'un liquide corporel qui est dégagé par le patient et qui doit être analysé puisse parvenir directement au dispositif d'analyse (4), mais que le liquide corporel arrivant sur le corps absorbant soit retenu par l'élément de séparation (8) et maintenu éloigné du dispositif d'analyse (4), et **en ce que** l'élément de séparation (8) est constitué par un insert (2) qui est disposé dans le corps absorbant et qui contient le dispositif d'analyse (4), l'insert (2) étant réalisé sous la forme d'une cuvette, et **en ce que** l'insert (2) est disposé substantiellement à affleurement avec la face supérieure (14) du corps absorbant qui est dirigée vers le corps du patient.

2. Article d'hygiène selon la revendication 1, **caractérisé en ce que** l'insert (2) comporte des parois latérales (10) qui forment un col en direction du corps du patient.

3. Article d'hygiène selon l'une quelconque des revendications précédentes, **caractérisé en ce que** les zones de bord (12) de l'insert (2) sont repliées sur la face supérieure de ce dernier.

4. Article d'hygiène selon l'une quelconque des revendications précédentes, **caractérisé en ce que** l'insert (2) est délimité par une feuille (6) imperméable aux liquides.

5. Article d'hygiène selon l'une quelconque des revendications précédentes, **caractérisé en ce que** le dispositif d'analyse (4) est disposé en reposant sur une face (20 ) de l'élément de séparation (8).

6. Article d'hygiène selon l'une quelconque des revendications précédentes, **caractérisé en ce que** le dispositif d'analyse (4) présente une unité d'indication optique.

7. Article d'hygiène selon la revendication 5 ou 6, **caractérisé en ce que** la face (20) de l'élément de séparation (8), sur laquelle repose le dispositif d'analyse (4), est réalisé sous une forme transparente au moins dans la zone du dispositif d'analyse (4) de telle manière que l'on puisse faire une lecture visuelle de l'unité d'indication.

8. Article d'hygiène selon l'une quelconque des revendications précédentes, **caractérisé en ce que** l'élément de séparation (8) qui contient le dispositif d'analyse (4) peut être séparé de l'article d'hygiène.

9. Article d'hygiène selon l'une quelconque des revendications précédentes, **caractérisé en ce que** l'élément de séparation (8) qui contient le dispositif d'analyse (4) est fixé de manière détachable sur l'article d'hygiène au moyen de moyens de fixation agissant par collage ou par adhésion.

10. Article d'hygiène selon l'une quelconque des revendications précédentes, **caractérisé en ce que** l'insert (2) qui contient le dispositif d'analyse comporte une couche de réception et de transport des liquides (18) qui conduit le liquide corporel au dispositif d'analyse.

11. Article d'hygiène selon la revendication 10, **caractérisé en ce que** la couche de réception et de transport des liquides (18) comprend des fibres de cellulose sans adjonction de matériaux polymères superabsorbants.
